# FASCICULE DE BREVET EUROPEEN

(11) **EP 1 616 281 B1**
(45) Date de publication et mention de la délivrance du brevet: **02.01.2019**
(21) Numéro de dépôt: 04742553.3
(22) Date de dépôt: 21.04.2004
(51) Int. Cl.: G06F 19/00, A61F 5/01

(54) **DISPOSITIF D'AIDE À LA SÉLECTION D'UNE ORTHÈSE DE CONTENTION ET À SON ADAPTATION À LA MORPHOLOGIE D'UN MEMBRE**
HILFSVORRICHTUNG ZUR AUSWAHL EINER KOMPRESSIONSORTHESE UND ANPASSUNG DER ORTHESE AN DIE MORPHOLOGIE EINES KÖRPERTEILS
DEVICE FOR ASSISTANCE IN THE SELECTION OF A COMPRESSION ORTHESIS AND IN ADAPTING SAME TO THE MORPHOLOGY OF A LIMB

(30) Priorité: 22.04.2003 FR 0304931
(43) Date de publication de la demande: 18.01.2006
(73) Titulaire: Laboratoires Innothera S.A.S., 94110 Arcueil (FR)
(72) Inventeur: BASSEZ, Sophie, 91140 Villebon sur Yvette (FR); TESTUD, Jean-Louis, 75013 Paris (FR)
(74) Mandataire: Nony
(86) Numéro de dépôt international: PCT/FR2004/000976
(87) Numéro de publication internationale: WO 2004/095342

(56) Documents cités:
- US-A1- 2002 010 408
- US-B1- 6 383 148
- CHU T -M ET AL: "Three-dimensional finite element stress analysis of the polypropylene, ankle-foot orthosis: static analysis" MEDICAL ENGINEERING & PHYSICS, JULY 1995, UK, vol. 17, no. 5, 1995, pages 372-379, XP002277428 ISSN: 1350-4533
- SYNGELLAKIS S ET AL: "Assessment of the non-linear behaviour of plastic ankle foot orthoses by the finite element method" PROCEEDINGS OF THE INSTITUTION OF MECHANICAL ENGINEERS, PART H (JOURNAL OF ENGINEERING IN MEDICINE), 2000, MECH. ENG. PUBLICATIONS, UK, vol. 214, no. H5, 2000, pages 527-539, XP001161162 ISSN: 0954-4119
- HANAFUSA A ET AL: "Computer assisted orthosis design system for malformed ears-automatic shape modification method for preventing excessive corrective force" PROCEEDINGS OF THE 22ND ANNUAL INTERNATIONAL CONFERENCE OF THE IEEE ENGINEERING IN MEDICINE AND BIOLOGY SOCIETY (CAT. NO.00CH37143), PROCEEDINGS OF THE 22ND ANNUAL INTERNATIONAL CONFERENCE OF THE IEEE ENGINEERING IN MEDICINE AND BIOLOGY SOCIETY, CHIC, 2000, pages 1976-1978 vol.3, XP002277429 2000, Piscataway, NJ, USA, IEEE, USA ISBN: 0-7803-6465-1
- BUCKLEY M A ET AL: "Computer simulation of the dynamics of a human arm and orthosis linkage mechanism" PROCEEDINGS OF THE INSTITUTION OF MECHANICAL ENGINEERS, PART H (JOURNAL OF ENGINEERING IN MEDICINE), 1997, MECH. ENG. PUBLICATIONS, UK, vol. 211, no. H5, 1997, pages 349-357, XP001161163 ISSN: 0954-4119

## Description

L'invention concerne un dispositif d'aide à la sélection d'une orthèse de contention et à son adaptation à la morphologie d'un membre auquel est destinée cette orthèse. L'invention concerne plus précisément les orthèses compressives tubulaires réalisées en matériaux textiles élastiques et tricotés.

Ces orthèses peuvent prendre plusieurs formes. Par exemple, s'agissant des orthèses compressives d'un ou des deux membres inférieurs, il peut s'agir de bas au sens strict (couvrant la cuisse et le jarret), de collants (couvrant les deux membres inférieurs et l'abdomen jusqu'à la ceinture, en une seule pièce), de mono-collants (collants munis d'une seule jambe, destinés à la contention d'un seul des membres inférieurs) ou encore de chaussettes (couvrant le jarret seul). Dans la suite, on utilisera le terme "bas" bien que l'invention ne soit pas limitée à un article particulier, mais s'applique aussi bien à toutes les orthèses compressives. L'invention peut également s'appliquer aux orthèses compressives destinées aux membres supérieurs.

Pour permettre une compression forte du ou des membres, ces orthèses sont réalisées en un matériau élastique, typiquement une maille tricotée de texture très serrée procurant l'effet thérapeutique recherché, à savoir une contention/compression à un degré thérapeutique, avec dégressivité à partir de la cheville.
Les bas de contention médicaux sont des bas qui exercent une pression, mesurée à la cheville, de 10 à plus de 36 mm Hg (13 à 48 hPa ; on utilisera toutefois dans la présente description le millimètre de mercure comme unité de mesure de pression compte tenu de son usage universel dans le domaine de la phlébologie et de la contention médicale).

L'une des difficultés, tout particulièrement avec les orthèses des classes les plus compressives, réside pour le praticien prescripteur dans le choix d'une taille et d'une classe d'orthèse la mieux adaptée à la pathologie du patient, c'est-à-dire procurant sur toute l'étendue du membre une contention ni trop faible, ni trop forte.

De plus, sauf à faire réaliser un bas sur mesure, le praticien doit choisir pour sa prescription une taille particulière dans des gammes dimensionnelles préexistantes, pour lesquelles les pressions appliquées par les différents articles de ces gammes sont établies par rapport à un gabarit dont la forme et les dimensions sont normalisées ("modèle Hohenstein").

La délivrance d'une taille d'orthèse se fait par prise de mesure de la jambe du patient à différentes hauteurs, par exemple trois mesures périmétriques à la cheville, au mollet et à la cuisse, ainsi que les hauteurs sol-genou et sol-entrejambe. A partir de ces mesures, le pharmacien ou l'orthopédiste détermine à partir d'un tableau ou d'une échelle la taille de l'article la mieux appropriée au patient. Cette manière de procéder est cependant assez empirique et, en tout état de cause, ne donne pas au médecin prescripteur une véritable idée du profil de pression qui sera réellement appliqué à la jambe, notamment pour les patients dont la morphologie de jambe est éloignée de la forme normalisée. Or la prescription d'une orthèse inappropriée peut se traduire localement par certaines zones de contention excessive ou, inversement, insuffisante.

L'article de Chu T.-M et all. « Three-dimensional finite element stress analysis of the polypropylene, ankle-foot orthosis : static analysis », Med. Eng. Phys, 17(5), pp. 372-379 (1995) décrit un procédé informatique pour étudier les contraintes exercées localement en différents points de la cheville et du pied par une orthèse semi-rigide destinée à maintenir et supporter cette région du membre, de façon passive, pour éviter des contraintes excessives lors de la marche. Dans le cas de la présente invention, il ne s'agit pas seulement de contenir le membre, mais de le comprimer de manière active, même au repos, pour produire un effet thérapeutique par amélioration du retour veineux.

L'un des buts de l'invention est de remédier à aux difficultés précitées en proposant un dispositif permettant au praticien d'évaluer l'adaptation de telle ou telle dimension d'orthèse compressive à la morphologie de la jambe d'un patient donné, de façon à pouvoir choisir en connaissance de cause celle qui est susceptible de procurer à ce patient l'effet thérapeutique optimal.

Un autre but de l'invention est de proposer un outil qui permette, dans le cadre d'une étude clinique d'une population de patients, de voir si l'échelonnement existant des différentes tailles d'un produit donné est bien adapté à la majorité de cette population ou si, au contraire, une gamme de tailles différentes serait mieux appropriée pour couvrir les besoins du plus grand nombre de patients.

À cet effet, le dispositif de l'invention comprend les moyens énoncés à la revendication 1 annexée ; les sous -revendications visent des mises en oeuvre préférentielles avantageuses.

On va maintenant décrire un exemple de mise en oeuvre de l'invention, en référence aux dessins annexés.
La figure 1 est une vue schématique montrant les différents moyens contribuant à la mise en oeuvre de l'invention.
La figure 2 illustre la modélisation de la jambe du patient.
La figure 3 illustre la modélisation de l'orthèse.
Les figures 4 à 7 montrent la manière dont il est possible d'afficher, sous forme graphique, les effets que l'orthèse est susceptible de produire sur la jambe du patient.

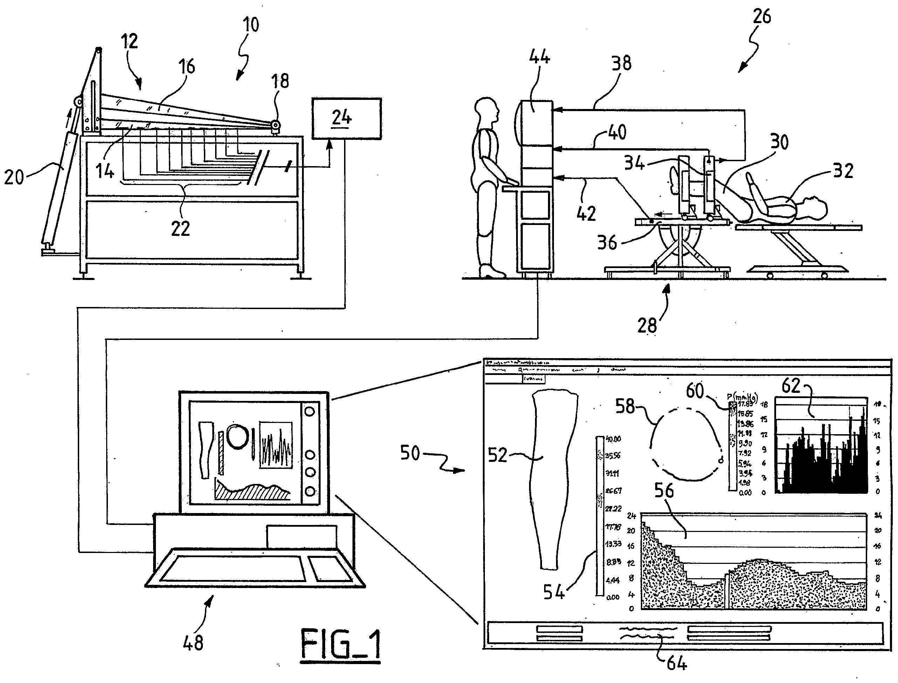

La figure 1 représente; de façon générale, les différents moyens permettant d'évaluer les effets de la contention d'une orthèse particulière sur une morphologie de jambe précise.

Il convient tout d'abord de modéliser, sous forme numérique, les caractéristiques dimensionnelles et rhéologiques de l'orthèse.

La loi rhéologique (c'est-à-dire la relation entre la tension appliquée au produit et la déformation radiale résultante) peut être en particulier déterminée au moyen d'un extensomètre 10 tel que celui décrit dans le WO-A-01/11337 (*Innothéra Topic International*)*.* Cet extensomètre comporte une forme 12 apte à recevoir une orthèse enfilée dessus, avec deux branches allongées 14, 16 articulées en 18 à la manière d'un compas. Les branches peuvent être écartées de manière contrôlée au moyen d'un vérin 20, et des capteurs 22 répartis sur la longueur d'une branche permettent de mesurer la tension radiale appliquée sur toute la longueur de l'orthèse sous la contrainte d'extension radiale appliquée par le vérin 20. Ces différentes mesures sont numérisées par un dispositif 24, qui produit une série
de données permettant d'évaluer, par modélisation et/ou interpolation, la loi rhéologique de l'orthèse sur toute la longueur de celle-ci.

En ce qui concerne la morphologie de la jambe, la forme de celle-ci peut être établie par divers moyens en eux-mêmes connus.

On peut par exemple utiliser une installation 26 telle que celle décrite dans les FR-A-2 774 276 et FR-A-2 804 595 (*Innothéra Topic International*), qui décrivent un pléthysmographe laser 28 permettant d'établir une cartographie très précise d'un membre 30 d'un sujet 32 le long de sections successives de ce membre. Le pléthysmographe 28 comporte une couronne de capteurs 34 permettant d'analyser par triangulation la forme d'une section de la jambe placée dans l'espace central de cette couronne. Cette dernière peut être déplacée en translation le long d'un axe linéaire 36, par pas successifs, pour réitérer la prise de mesure pour différentes sections sur toute la longueur du membre 30. Les signaux de mesure, ainsi que les positions de l'axe circulaire et de l'axe linéaire sont transmis par des liaisons 38, 40, 42 à un dispositif 44 permettant de reconstituer à partir de ces informations une représentation tridimensionnelle de la jambe 30 sous forme d'un ensemble de courbes paramétrées régulièrement espacées.

Après avoir ainsi défini les caractéristiques dimensionnelles et rhéologiques de l'orthèse et les caractéristiques morphologiques du membre, les informations correspondantes sont mémorisées dans des fichiers de données respectifs d'un calculateur 48, données qui sont ensuite confrontées de manière à déterminer les valeurs de pression de contention susceptibles d'être exercées sur le membre.

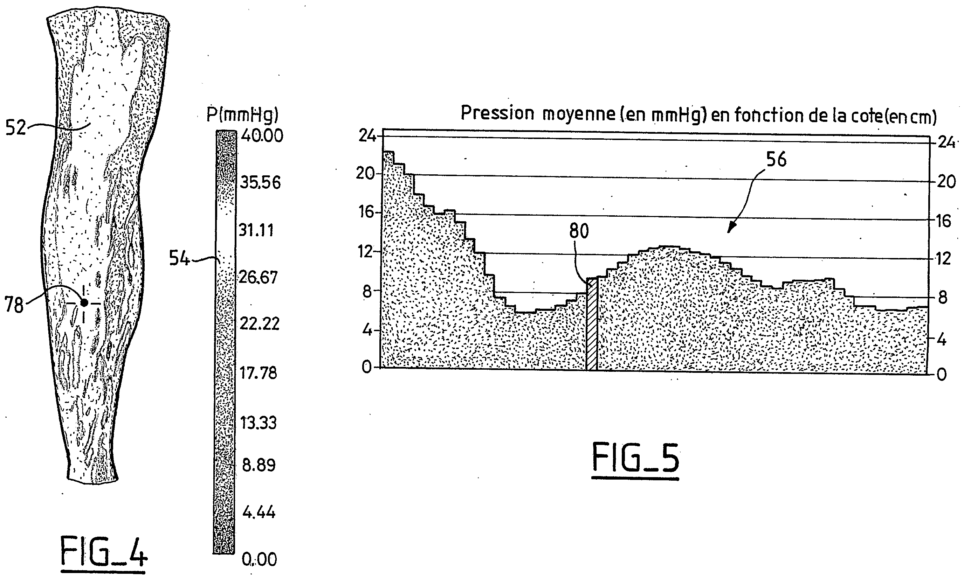

Ces valeurs de pression sont avantageusement présentées au praticien sous la forme d'un affichage graphique 50 comportant par exemple (les différents éléments de cet affichage seront décrits plus en détail en référence aux figures 4 à 8) :
- une vue tridimensionnelle 52 de la jambe, où les pressions sont codées en niveaux de gris ou en fausses couleurs, repérés par une échelle de pressions 54,
- une caractéristique 56 donnant la variation de la pression moyenne, section par section, sur la longueur de la jambe depuis la cheville jusqu'à la cuisse,
- une représentation tridimensionnelle 58 d'une section de jambe à un niveau choisi par le praticien, également codée en niveaux de gris ou en fausses couleurs avec une échelle correspondante 60,
- une caractéristique 62 donnant la répartition angulaire des pressions sur la section représentée en 58,
- une zone d'informations numériques 64 correspondant à certains points particuliers désignés sur les représentations 52 ou 58.

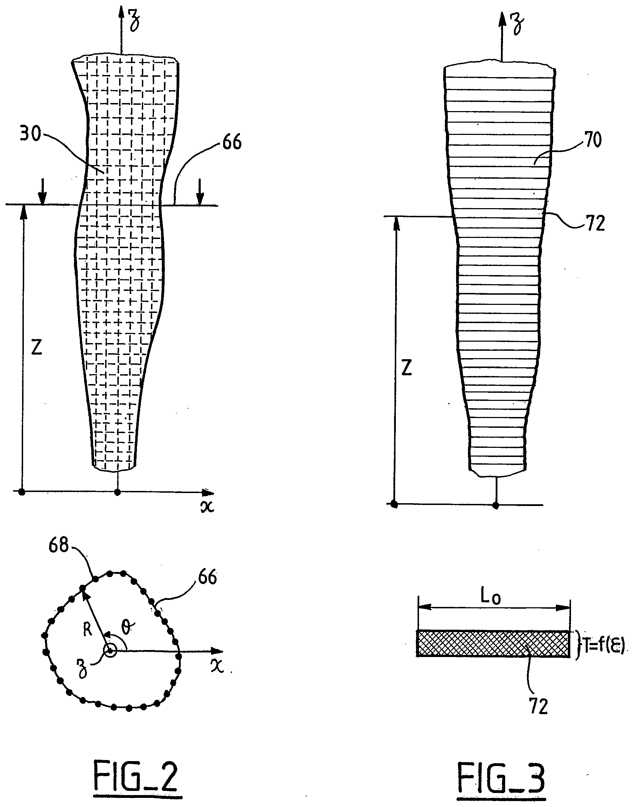

On va maintenant décrire, en référence aux figures 2 et 3, la manière dont sont constitués les fichiers de données représentatives, respectivement, des caractéristiques morphologiques du membre et des caractéristiques dimensionnelles et rhéologiques de l'orthèse.

En ce qui concerne le membre 30 (figure 2) les données sont définies pour une série de sections 66 à des cotes *Z* successives, ces données étant par exemple déterminées par la position de points 68 définis par leur cote *Z* et leurs coordonnées polaires *R*, *θ* par rapport à un référentiel (*Ox,Oz*). Les cotes de mesure doivent au minimum comprendre les cotes correspondant aux hauteurs normalisées *b, b1, c, d, e, f* et *g* d'une jambe Hohenstein, l'origine du repère dans la direction *z* étant *Z* = 0 au sol, de manière à disposer d'une référence verticale commune à la jambe et à l'orthèse.

Selon la précision des mesures recherchée, on réalisera ainsi un maillage plus ou fin de la jambe, où chaque noeud du maillage est repéré par ses coordonnées *Z, r, θ.*

L'étape suivante consiste, à partir de ces données de position des différents points du maillage, à déterminer pour chaque section le périmètre *C* et la courbure *c* en tout point de cette section à partir des coordonnées des points du contour, par des méthodes en elles-mêmes connues.

En ce qui concerne l'orthèse 70 (figure 3), il convient tout d'abord d'en déterminer les caractéristiques dimensionnelles.

S'agissant d'un produit qui peut être mis à plat, on utilise avantageusement la largeur à plat au repos *L₀* du produit à différentes cotes *Z* définissant une pluralité de niveaux de mesure 72. Les points de mesure, au minimum ceux correspondant aux hauteurs normalisées *b, b1, c, d, e, f* et *g* d'une jambe Hohenstein, sont repérés sur l'orthèse 70 par rapport au talon (origine des cotes *Z* = 0 au niveau du sol).

Les caractéristiques rhéologiques, c'est-à-dire la loi donnant la déformation longitudinale *ε* en fonction de la tension appliquée *T,* peuvent être déterminées soit par des mesures dynamométriques soit, comme exposé en référence à la figure 1, avec un extensomètre dans les conditions précédemment définies, l'élongation *ε* étant calculée comme étant le rapport de la longueur d'application sur la longueur de repos. À partir de ces mesures on extrapole une loi permettant de déterminer en tout point de l'orthèse la tension en fonction de la déformation.

À partir des données ainsi recueillies et mémorisées, le dispositif calcule ensuite la pression de contention que l'orthèse 70 exercerait sur la jambe 30, telle que modélisée, si elle était enfilée sur cette dernière. Cette pression de contention est calculée pour chaque section de la jambe (section associée à une cote *Z* par rapport au sol), sur les différents points du contour de cette section. Le principe de calcul de la pression de contention en un point donné repose sur l'application de la loi de Laplace *P* = *T.c*, où *T* représente la tension linéique du textile dans le sens circonférentiel et c la courbure de la jambe sur laquelle est appliquée la contention.

On a ainsi réalisé une cartographie tridimensionnelle simulant les pressions de contention qu'appliquerait l'orthèse à la surface de la jambe si elle était enfilée sur cette dernière.

Cette cartographie peut avantageusement être visualisée sous forme graphique de différentes manières, comme on va l'exposer en référence aux figures 4 à 8 qui représentent divers affichages présentés au praticien pour l'aider dans sa prescription ou dans son étude clinique.

Il est notamment possible d'afficher (figure 4) une représentation tridimensionnelle 52 de la jambe, avec pour le praticien possibilité de faire tourner la jambe, sélectionner un point, zoomer sur une partie de cette jambe, etc. La représentation 52 est avantageusement codée en niveaux de gris ou en fausses couleurs, avec une échelle de référence 54 permettant d'évaluer le niveau de la pression appliquée (par exemple bleu pour une pression faible, vert pour une pression moyenne, jaune pour une pression forte, rouge pour une pression très forte). L'échelle de pression 54 s'étend du minimum au maximum des valeurs calculées, mais il est possible de la modifier pour détailler de façon plus fine une zone de pression particulière.

Le praticien peut également désigner un point particulier 78, par exemple au moyen d'une souris et d'un interface graphique, afin d'afficher dans une zone de valeurs 64 (figure 1) les valeurs numériques associés à ce point précis : cote, courbure, pression, etc.

Par ailleurs, le dispositif calcule également les valeurs des pressions moyennes exercées sur les différents points d'un même contour à une cote donnée, ce qui permet d'afficher un profil de pression 56 en fonction de la cote (figure 5). La désignation du point 78 sur la représentation de la figure 4 est reportée en 80 sur le profil de la figure 5, par exemple par une zone de couleur différente, visualisant de façon immédiate le niveau de pression correspondant à la cote du point 78.

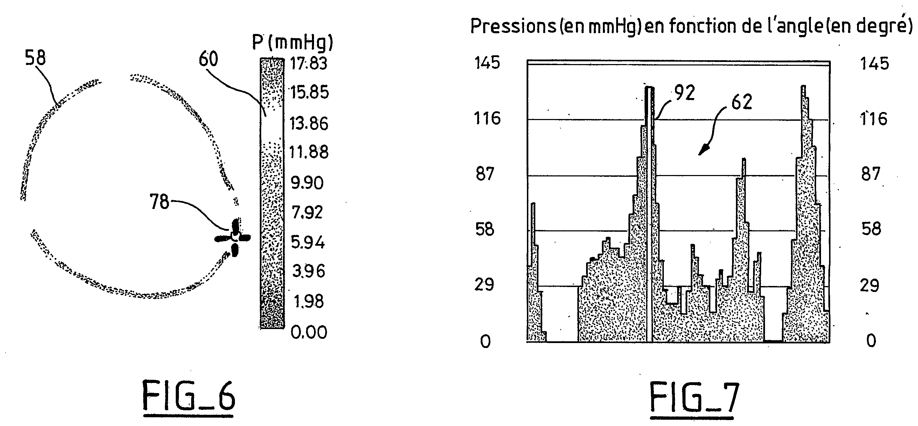

Pour évaluer la répartition des pressions exercée aux différents points d'une section donnée, le praticien dispose également d'une visualisation bidimensionnelle (figure 6) représentant une coupe 58 de la jambe suivant une cote constante. Ici encore, les niveaux de pression sont codés au moyen d'une échelle de gris ou de fausses couleurs 60 et le point 78 désigné sur la représentation de la figure 4 est également repris sur cette vue bidimensionnelle (les différents affichages sont tous interdépendants, de sorte que toute modification de la cote du point 78 par l'opérateur modifiera automatiquement le niveau de la coupe 58).

Par ailleurs, un graphique 62 (figure 7) donne la variation en fonction de l'angle de la pression sur la section de jambe illustrée figure 6 : chaque barre du graphique 62 représente un point de la section, le point sélectionné en 78 étant représente par une barre 92 de couleur différente. Enfin, de façon générale, il est possible d'opérer une sélection partielle en définissant une zone angulaire d'intérêt. Le secteur angulaire de la jambe non sélectionné pourra être visualisé d'une manière particulière sur les affichages, par exemple par une zone grisée.

## Revendications

1. Un dispositif d'aide à la sélection d'une orthèse de contention réalisée en un matériau élastique et procurant une contention/compression à un degré thérapeutique, avec dégressivité à partir de la cheville, et
à son adaptation à la morphologie d'un membre auquel est destinée cette orthèse, **caractérisé en ce qu'**il comprend :
- des moyens (26) pour établir un premier fichier de données représentatives des caractéristiques morphologiques du membre (30), ce premier fichier de données comprenant les coordonnées, dans un espace tridimensionnel, d'un maillage de points (68) répartis à la surface du membre le long d'une succession de contours (66) définis à différentes cotes successives (Z) de ce membre ;
- des moyens (10) pour établir un second fichier de données représentatives des caractéristiques dimensionnelles et rhéologiques de l'orthèse définies à différentes cotes successives (Z) de cette orthèse ;
- des moyens (48) de simulation de contention, aptes à déterminer, à partir des données des premier et second fichiers, des valeurs de pressions de contention susceptibles d'être exercées par l'orthèse sur le membre en une pluralité de points dudit maillage ; et
- des moyens (50) de présentation desdites valeurs de pression déterminées par les moyens de simulation de contention.

2. Le dispositif de la revendication 1, dans lequel le second fichier de données contient des données de largeur à plat (Lₒ) de l'orthèse auxdites cotes successives, et des données (Δx/Δf) représentatives de la caractéristique de déformation de l'orthèse en fonction de la tension exercée sur celle-ci entre des points situés à des cotes consécutives.

3. Le dispositif selon l'une quelconque des revendications précédentes, dans lequel l'orthèse est un bas de contention/compression médical exerçant une pression, mesurée à la cheville, de 13 à 48 hPa.

4. Le dispositif selon l'une quelconque des revendications précédentes, dans lequel
- le premier fichier de données comprend les coordonnées, dans un espace tridimensionnel, d'un maillage de points (68) répartis à la surface du membre le long d'une succession de contours (66) définis à différentes cotes successives (Z) de ce membre, les cotes de mesure comprenant au minimum les cotes correspondant aux hauteurs normalisées b, b1, c, d, e, f et g d'une jambe Hohenstein, l'origine du repère dans la direction z étant Z = 0 au sol, et/ou
- le second fichier de données comprend des données représentatives des caractéristiques dimensionnelles et rhéologiques de l'orthèse définies à différentes cotes successives (Z) de cette orthèse, les points de mesure comprenant, au minimum, ceux correspondant aux hauteurs normalisées b, b1, c, d, e, f et g d'une jambe Hohenstein, et étant repérés sur l'orthèse par rapport au talon, l'origine des cotes Z = 0 étant au niveau du sol.

5. Le dispositif selon l'une quelconque des revendications précédentes, dans lequel les moyens pour établir le premier fichier comportent un pléthysmographe et/ou les moyens pour établir le second fichier comportent un extensiomètre.

6. Le dispositif de la revendication 1, comprenant en outre des moyens de désignation, aptes à permettre la désignation d'un point du maillage par un opérateur du dispositif, et à commander les moyens de présentation des valeurs de pression pour afficher la valeur de la pression calculée au point (82) ainsi désigné.

7. Le dispositif de la revendication 1, comprenant en outre des moyens de désignation, aptes à permettre la désignation d'une cote du maillage par un opérateur du dispositif, et à commander les moyens de présentation des valeurs de pression pour afficher la valeur des pressions calculées aux différents points du contour de la section du membre située à la cote (78) ainsi désignée.

8. Le dispositif de la revendication 1, dans lequel les moyens de présentation comprennent des moyens graphiques aptes à afficher une représentation graphique tridimensionnelle (52) du membre et à associer localement à cette représentation graphique les valeurs de pression calculées aux différents points dudit maillage.

9. Le dispositif de la revendication 1, dans lequel les moyens de présentation comprennent des moyens graphiques aptes à afficher une représentation graphique bidimensionnelle (58) d'une section du membre et à associer localement à cette représentation graphique les valeurs de pression calculées aux différents points du contour de cette section.

10. Le dispositif de la revendication 8 ou 9, dans lequel les moyens graphiques associent les valeurs de pression calculées à la représentation graphique par superposition à cette représentation graphique, à l'endroit des différents points, d'un codage en fausses couleurs ou en niveaux de gris de la pression calculée en ces points.

11. Le dispositif de la revendication 1, dans lequel les moyens de présentation comprennent des moyens graphiques aptes à afficher une caractéristique (62) donnant la variation, en fonction de la position angulaire, de la pression calculée aux différents points du contour d'une section du membre située à une cote donnée.

12. Le dispositif de la revendication 1, dans lequel:
- les moyens de simulation sont également aptes à déterminer des valeurs moyennes de la pression de contention aux points situés à une même cote, et
- les moyens de présentation comprennent des moyens graphiques aptes à afficher une caractéristique (56; 80) donnant la variation, en fonction de la cote, de la pression moyenne de contention ainsi calculée.

13. Procédé d'aide à la sélection d'une orthèse de contention réalisée en un matériau élastique procurant une contention/compression à un degré thérapeutique, avec dégressivité à partir de la cheville, et à son adaptation à la morphologie d'une jambe auquel est destinée cette orthèse, ledit procédé comprenant les étapes suivantes :
- établissement d'un premier fichier de données représentatives des caractéristiques morphologiques de la jambe, ce premier fichier de données comprenant les coordonnées, dans un espace tridimensionnel, d'un maillage de points répartis à la surface de la jambe le long d'une succession de contours définis à différentes cotes successives de cette jambe ;
- établissement d'un second fichier de données représentatives des caractéristiques dimensionnelles et rhéologiques de l'orthèse définie à différentes cotes successives de cette orthèse ;
- détermination, à partir des données et premier et second fichier, des valeurs de pression de contention susceptibles d'être exercées par l'orthèse sur la jambe en une pluralité de points dudit maillage ;
- présentation desdites valeurs de pression déterminées à l'étape précédente.

14. Procédé selon la revendication immédiatement précédente, dans lequel on évalue, à partir de la présentation desdites valeurs de pression l'adaptation d'une dimension de l'orthèse à la morphologie de ladite jambe.

15. Procédé selon l'une quelconque des deux revendications immédiatement précédentes, mettant en oeuvre un dispositif selon l'une quelconque des revendications 1 à 12.

## Patentansprüche

1. Vorrichtung zur Auswahl einer Stützorthese, die aus einem elastischen Material hergestellt ist und eine Stützung/Kompression mit therapeutischem Grad vom Knöchel aus abnehmend bietet, und zur Anpassung an die Morphologie einer Gliedmaße, für die diese Orthese bestimmt ist,
**dadurch gekennzeichnet, dass** sie umfasst:
- Mittel (26), um eine erste Datei von Daten zu erstellen, die für die morphologischen Merkmale der Gliedmaße (30) repräsentativ sind, wobei diese erste Datendatei die Koordinaten eines Netzes von Punkten (68) in einem dreidimensionalen Raum umfasst, die an der Oberfläche der Gliedmaße entlang einer Aufeinanderfolge von Konturen (66), die an verschiedenen aufeinanderfolgenden Maßen (Z) dieser Gliedmaße definiert sind, verteilt sind;
- Mittel (10), um eine zweite Datei von Daten zu erstellen, die für die dimensionalen und rheologischen Merkmale der Orthese repräsentativ sind, die an verschiedenen aufeinanderfolgenden Maßen (Z) dieser Orthese definiert sind;
- Mittel (48) zur Stützstimulation, die geeignet sind, auf Basis der Daten der ersten und zweiten Dateien Stützdruckwerte zu bestimmen, die von der Orthese auf die Gliedmaße an einer Vielzahl von Punkten des Netzes ausgeübt werden können; und
- Mittel (50) zur Darstellung der von den Stützsimulationsmitteln bestimmten Druckwerte.

2. Vorrichtung nach Anspruch 1, bei der die zweite Datendatei Daten mit einer Liegebreite (Lₒ) der Orthese an den aufeinanderfolgenden Maßen und Daten (Δx/Δf) enthält, die für die Verformungscharakteristik der Orthese in Abhängigkeit von der Spannung, die auf diese zwischen Punkten, die sich an aufeinanderfolgenden Maßen befinden, ausgeübt wird, repräsentativ sind.

3. Vorrichtung nach einem der vorhergehenden Ansprüche, bei der die Orthese ein medizinischer Stütz-/Kompressionsstrumpf ist, der einen Druck, gemessen am Knöchel, von 13 bis 48 hPa ausübt.

4. Vorrichtung nach einem der vorhergehenden Ansprüche, bei der
- die erste Datendatei die Koordinaten eines Netzes von Punkten (68) in einem dreidimensionalen Raum umfasst, die an der Oberfläche der Gliedmaße entlang einer Aufeinanderfolge von Konturen (66) verteilt sind, die an verschiedenen aufeinanderfolgenden Maßen (Z) dieser Gliedmaße definiert sind, wobei die Maße mindestens die Maße entsprechend den Normhöhen b, b1, c, d, e, f und g eines Beins Hohenstein umfassen, wobei der Ursprung des Bezugspunktes in z-Richtung Z = 0 am Boden ist, und/oder
- die zweite Datendatei Daten umfasst, die für die dimensionalen und rheologischen Merkmale der Orthese repräsentativ sind, die an verschiedenen aufeinanderfolgenden Maßen (Z) dieser Orthese definiert sind, wobei die Messpunkte mindestens jene umfassen, die den Normhöhen b, b1, c, d, e, f und g eines Beins Hohenstein entsprechen, und auf der Orthese in Bezug zur Ferse bezeichnet sind, wobei der Ursprung der Maße Z = 0 auf Bodenniveau ist.

5. Vorrichtung nach einem der vorhergehenden Ansprüche, bei der die Mittel, um die erste Datei zu erstellen, einen Plethysmographen umfassen, und/oder die Mittel, um die zweite Datei zu erstellen, ein Extensometer umfassen.

6. Vorrichtung nach Anspruch 1, ferner umfassend Bezeichnungsmittel, die geeignet sind, die Bezeichnung eines Punktes des Netzes durch einen Bediener der Vorrichtung zu ermöglichen, und die Mittel zur Darstellung der Druckwerte zu steuern, um den Wert des an dem so bezeichneten Punkt (82) berechneten Drucks anzuzeigen.

7. Vorrichtung nach Anspruch 1, ferner umfassend Bezeichnungsmittel, die geeignet sind, die Bezeichnung eines Maßes des Netzes durch einen Bediener der Vorrichtung zu ermöglichen, und die Mittel zur Darstellung der Druckwerte zu steuern, um die berechneten Druckwerte an den verschiedenen Punkten der Kontur des Abschnitts der Gliedmaße, der sich an dem so bezeichneten Maß (78) befindet, anzuzeigen.

8. Vorrichtung nach Anspruch 1, bei der die Darstellungsmittel graphische Mittel umfassen, die geeignet sind, eine dreidimensionale graphische Darstellung (52) der Gliedmaße anzuzeigen und lokal dieser graphischen Darstellung die an den verschiedenen Punkten des Netzes berechneten Druckwerte zuzuordnen.

9. Vorrichtung nach Anspruch 1, bei der die Darstellungsmittel graphische Mittel umfasst, die geeignet sind, eine zweidimensionale graphische Darstellung (58) eines Abschnitts der Gliedmaße anzuzeigen und lokal dieser graphischen Darstellung die an den verschiedenen Punkten der Kontur dieses Abschnitts berechneten Druckwerte zuzuordnen.

10. Vorrichtung nach Anspruch 8 oder 9, bei der die graphischen Mittel die berechneten Druckwerte der graphischen Darstellung durch Überlagerung einer Codierung in kontrastierenden Farben oder Graustufen des an diesen Punkten berechneten Drucks über diese graphische Darstellung an der Stelle der verschiedenen Punkte.

11. Vorrichtung nach Anspruch 1, bei der die Darstellungsmittel graphische Mittel umfassen, die geeignet sind, ein Merkmal (62) anzuzeigen, das in Abhängigkeit von der Winkelposition die Variation des Drucks, der an den verschiedenen Punkten der Kontur eines Abschnitts der Gliedmaße, der sich an einem gegebenen Maße befindet, angibt.

12. Vorrichtung nach Anspruch 1, bei der:
- die Simulationsmittel auch geeignet sind, Durchschnittswerte des Stützdrucks an den an einem selben Maß befindlichen Punkten zu bestimmen, und
- die Darstellungsmittel graphische Mittel umfassen, die geeignet sind, ein Merkmal (56; 80) anzuzeigen, das in Abhängigkeit von dem Maß den so berechneten durchschnittlichen Stützdruck angibt.

13. Verfahren zur Auswahl einer Stützorthese, die aus einem elastischen Material hergestellt ist und eine Stützung/Kompression mit therapeutischem Grad vom Knöchel aus abnehmend bietet, und zur Anpassung an die Morphologie eines Beins, für das diese Orthese bestimmt ist, wobei das Verfahren die folgenden Schritte umfasst:
- Erstellung einer ersten Datei von Daten, die für die morphologischen Merkmale des Beins repräsentativ sind, wobei diese erste Datendatei die Koordinaten eines Netzes von Punkten in einem dreidimensionalen Raum umfasst, die an der Oberfläche des Beins entlang einer Aufeinanderfolge von Konturen, die an verschiedenen aufeinanderfolgenden Maßen dieses Beins definiert sind, verteilt sind;
- Erstellung einer zweiten Datei von Daten, die für die dimensionalen und rheologischen Merkmale der Orthese repräsentativ sind, die an verschiedenen aufeinanderfolgenden Maßen dieser Orthese definiert sind;
- auf Basis der Daten und der ersten und zweiten Datei Bestimmung der Stützdruckwerte, die von der Orthese auf das Bein an einer Vielzahl von Punkten des Netzes ausgeübt werden können;
- Darstellung der im vorhergehenden Schritt bestimmten Druckwerte.

14. Verfahren nach dem unmittelbar vorhergehenden Anspruch, bei dem auf Basis der Darstellung der Druckwerte die Anpassung einer Dimension der Orthese an die Morphologie des Beins bewertet wird.

15. Verfahren nach einem der zwei unmittelbar vorhergehenden Ansprüche, das eine Vorrichtung nach einem der Ansprüche 1 bis 12 einsetzt.

## Claims

1. Device for assistance in the selection of a compression orthosis made of an elastic material and providing compression to a therapeutic degree, with the compression decreasing from the ankle, and
in its adaptation to the morphology of a limb for which this orthosis is intended,
**characterized in that** it comprises:
- means (26) for establishing a first file containing data that are representative of the morphological characteristics of the limb (30), this first data file comprising the coordinates, in a three-dimensional space, of an array of points (68) distributed on the surface of the limb along a succession of defined contours (66) at different successive heights (Z) of this limb;
- means (10) for establishing a second file containing data that are representative of the dimensional and rheological characteristics of the orthosis defined at different successive heights (Z) of this orthosis;
- compression simulation means (48) which are able to determine, from the data of the first and second files, compression pressure values that are capable of being exerted by the orthosis on the limb at a plurality of points of said array; and
- means (50) for displaying said pressure values determined by the compression simulation means.

2. Device according to Claim 1, in which the second data file contains data for the flat width (L₀) of the orthosis at said successive heights, and data (Δx/Δf) that are representative of the deformation characteristic of the orthosis as a function of the tension exerted on the latter between points situated at consecutive heights.

3. Device according to either of the preceding claims, in which the orthosis is a medical compression stocking exerting a pressure of 13 to 48 hPa, measured at the ankle.

4. Device according to any one of the preceding claims, in which
- the first data file comprises the coordinates, in a three-dimensional space, of an array of points (68) distributed on the surface of the limb along a succession of defined contours (66) at different successive heights (Z) of this limb, the measurement heights comprising at a minimum the heights corresponding to the standardized heights b, b1, c, d, e, f and g of a Hohenstein leg, the origin of the reference frame in the z direction being Z = 0 at floor level; and/or
- the second data file comprises data that are representative of the dimensional and rheological characteristics of the orthosis defined at different successive heights (Z) of this orthosis, the measurement points comprising, at a minimum, those corresponding to the standardized heights b, b1, c, d, e, f and g of a Hohenstein leg, and being plotted on the orthosis with respect to the heel, the origin of the heights Z = 0 being at floor level.

5. Device according to any one of the preceding claims, in which the means for establishing the first file include a plethysmograph and/or the means for establishing the second file include an extensometer.

6. Device according to Claim 1, moreover comprising designation means which allow an operator of the device to designate a point of the array and which are able to command the pressure value display means to display the value of the pressure calculated at the point (82) thus designated.

7. Device according to Claim 1, moreover comprising designation means which allow an operator of the device to designate a height of the array and which are able to command the pressure value display means to display the value of the pressures calculated at the different points of the contour of the section of the limb situated at the height (78) thus designated.

8. Device according to Claim 1, in which the display means comprise graphical means for displaying a three-dimensional graphical representation (52) of the limb and for assigning locally to this graphical representation the pressure values calculated at the different points of said array.

9. Device according to Claim 1, in which the display means comprise graphical means for displaying a two-dimensional graphical representation (58) of a section of the limb and for assigning locally to this graphical representation the pressure values calculated at the different points of the contour of this section.

10. Device according to Claim 8 or 9, in which the graphical means assign the calculated pressure values to the graphical representation by overlaying this graphical representation, at the location of the different points, with a false-colour or grey-level coding of the pressure calculated at these points.

11. Device according to Claim 1, in which the display means comprise graphical means for displaying a characteristic (62) giving the variation, as a function of the angular position, of the pressure calculated at the different points of the contour of a section of the limb situated at a given height.

12. Device according to Claim 1, in which:
- the simulation means are likewise able to determine mean values of the compression pressure at the points situated at the same height, and
- the display means comprise graphical means for displaying a characteristic (56; 80) giving the variation, as a function of the height, of the mean compression pressure thus calculated.

13. Method for assistance in the selection of a compression orthosis made of an elastic material providing compression to a therapeutic degree, with the compression decreasing from the ankle, and in its adaptation to the morphology of a leg for which this orthosis is intended, said method comprising the following steps:
- establishing a first file containing data that are representative of the morphological characteristics of the leg, this first data file comprising the coordinates, in a three-dimensional space, of an array of points distributed on the surface of the leg along a succession of defined contours at different successive heights of this leg;
- establishing a second file containing data that are representative of the dimensional and rheological characteristics of the orthosis defined at different successive heights of this orthosis;
- determining, from the data and first and second file, compression pressure values that are capable of being exerted by the orthosis on the leg at a plurality of points of said array;
- displaying said pressure values determined at the preceding step.

14. Method according to the immediately preceding claim, in which the display of said pressure values is used to evaluate the adaptation of a dimension of the orthosis to the morphology of said leg.

15. Method according to either of the two immediately preceding claims, using a device according to any one of Claims 1 to 12.
